# EUROPEAN PATENT SPECIFICATION

(11) **EP 2 666 469 B1**
(45) Date of publication and mention of the grant of the patent: **07.12.2016**
(21) Application number: 11856355.0
(22) Date of filing: 15.07.2011
(51) Int. Cl.: A61K 36/8945, A61K 36/73, A61K 36/71, A61K 36/534, A61K 36/53, A61K 36/484, A61K 36/258, A61K 36/185, A61K 36/076, A61P 17/00, C12P 1/00

(54) **PHARMACEUTICAL COMPOSITION FOR TREATING MELASMA AND PREPARATION METHOD THEREFOR**
PHARMAZEUTISCHE ZUSAMMENSETZUNG ZUR BEHANDLUNG VON MELASMA UND HERSTELLUNGSVERFAHREN DAFÜR
COMPOSITION PHARMACEUTIQUE POUR TRAITER LE MÉLASMA ET SON PROCÉDÉ DE PRÉPARATION

(30) Priority: 21.01.2011 CN 201110023840
(43) Date of publication of application: 27.11.2013
(73) Proprietor: Jilin Zixin Pharmaceutical Research Institution LLC, Jilin 130041 (CN)
(72) Inventor: SUN, Dejun, Changchun Jilin 130041 (CN); YIN, Jinlong, Changchun Jilin 130041 (CN); SUN, Miaonan, Changchun Jilin 130041 (CN); ZHAO, Yizhuo, Changchun Jilin 130041 (CN); GUO, Chunsheng, Changchun Jilin 130041 (CN); GAO, Yanhui, Changchun Jilin 130041 (CN); LI, Xue, Changchun Jilin 130041 (CN)
(74) Representative: Care, Alison
(86) International application number: PCT/CN2011/077177
(87) International publication number: WO 2012/097579

(56) References cited:
- CN-A- 1 943 683
- CN-A- 101 366 940
- CN-A- 101 579 500
- CN-A- 101 732 683
- CN-A- 102 078 486
- US-A1- 2003 235 559

## Description

### Background of the Present Invention

### Field of Invention

The present invention relates to a pharmaceutical composition for treating melasma and a preparation method thereof.

### Description of Related Arts

Melasma does harm to a lot of women. The melasma is mainly caused by female endocrine disorders, mental stress, various diseases (liver and kidney function insufficiency, gynecological disease, diabetes, etc) as well as lack of vitamins and external chemical stimulation. For treating the melasma on skin, prevention combined with treatments is mainly provided. Conditioning female endocrine environment, keeping in a good mood, and actively preventing the gynecological diseases are effective methods for preventing the melasma. Specialized medicine such as the effective Grace Age activating protein is also utilized for treating the melasma.

Ginseng: a root of *panax* of the family *Araliaceae.* More than 30 kinds of ginseng saponins can be separated out from the ginseng (the ginseng saponins can be divided into three groups: oleanolic acid group, protopanaxdiol group and protopanaxatriol group), and are called ginsenoside -RX (wherein X = 0, a1, a2, a3, b1, b2, b3, c, d, e, f, g1, g2, g3, h1, h2, h3, s1, and s2); furthermore, pseudoginsenoside saponin F11 can also be separated out.

Peony: a root of *paeonia lactiflora* of the family *Paeoniaceas* of the Class *Dicotyledoneae.* The peony tastes bitter and sour, and can benefit liver and spleen diseases. The peony cleans blood, nourishes liver, relieves rheumatic symptoms, reduces pain, and arrests sweating. The peony cures thoracic and abdominal flank pain, diarrhea, abdominal pain, spontaneous sweating, cold, fever, irregular menstruation, uterine bleeding and vaginal discharge.

Poria: parasitic fungi on pine roots. The poria tastes sweet and light, has no side effects and benefits heart, lung, spleen, and kidney. The poria also induces diuresis, benefits spleen and stomach, and calms nerve.

Licorice: a root or rhizome of *glycyrrhiza uralensis* of the family *Fabaceae* of the Class *Dicotyledoneae.* The licorice has no side effects, tastes sweet and benefits viscera. The licorice has detoxification, expectorant, analgesic, antispasmodic as well as anti-cancer pharmacological effects. The licorice can be utilized for treating weak stomach and spleen, fatigue, heart palpitations, shortness of breath, cough, sputum, abdominal pain, limbs twin urgent pain, carbuncle swollen sore and drug toxicity.

Yam: a root of *dioscorea opposite.* The yam is sweet, warm, has no side effects and no toxic, benefits lung, spleen and kidney, and doesn't cause dryness-heat; the yam is good to spleen and stomach, strengthens kidney, vigor, ears and eyes, viscera as well as bones, calms nerve and has anti-aging effects. The yam is mainly utilized for weak spleen and stomach, fatigue, weakness, loss of appetite, diarrhea, weak lung, sputum, cough, weak kidney, weak waist, weak limb, frequent urination, nocturnal emission, premature ejaculation, whitish vaginal discharge, red skin, swelling, obesity and other illnesses.

Mint: an aboveground part of perennial herb of the family *Lamiaceae* of the genus *Mentha,* the mint is spicy and cool, benefits lung and liver, relieves rheumatism as well as heat, and cleans toxic. The mint cures cold, headache, red eyes, sore throat, indigestion, mouth sores, toothache, scabies and urticaria.

Salvia: a dried root or rhizome of *salvia miltiorrhiza* of the family *Lamiaceae* of the Class *Dicotyledoneae.* The salvia is bitter and slightly cold. And the salvia benefits heart and liver, invigorates the circulation of blood, regulates menstruation, decreases blood stasis and pain, removes carbuncle, calms nerves, and nourishes blood. The salvia also cures irregular menstruation, amenorrhea, dysmenorrhea, abdominal distension, chest and abdomen pain, arthralgia, sore throat, upset, insomnia; hepatosplenomegaly and angina.

Peach seed: a seed of peach of the rosids plant. The peach seed is bitter, sweet, and has no side effects. And the peach seed benefits heart, liver, and intestine. The peach seed also cures blood stasis, astriction, cough, asthma, amenorrhea, dysmenorrhea, abdominal distension, and dryness of intestine.

Orange peel: peels of orange of the family *Rutaceae.* The orange peel is spicy, bitter and warm. And the orange peel benefits spleen and lung. The orange peel also treats poor immunity, sputum, dyspepsia, abdominal distention, vomiting, chest tightness due to dampness and weak breath, poor appetite, and loose stools.

Penthorum chienese: a dried aboveground part of *penthorum chinense pursh* of the family *Saxifragaceae* of the genus *Penthorum.* The penthorum chienese is warm, sweet, and non-toxic, and the penthorum chienese has clearing heat, diuretic and urinary elimination, detoxification, blood circulation, strengthening liver and spleen, removing melasma and other effects. The penthorum chienese is mainly utilized for treating melasma, edema, amenorrhea, vaginal bleeding, vaginal discharge, bruises, as well as various types of hepatitis, cholecystitis and fatty liver.

### Summary of the Present Invention

An object of the present invention is to provide a pharmaceutical composition for treating melasma and a preparation method thereof.

Accordingly, in order to accomplish the above object, the present invention provides a preparation method of a pharmaceutical composition for treating melasma, as defined in the claims. A method is described comprising:
mixing the following four substances for obtaining a mixture, wherein the mixture is the pharmaceutical composition for treating the melasma, wherein the four substances comprises:
   A) ethanol extract of ginseng and salvia; B) volatile oil of mint and orange peel; C) aqueous extract of the ginseng, the salvia, the mint, the orange peel, peony, poria, licorice, peach seed, yam and penthorum chienese; D) a fermentation conversion solution of fruits and vegetables;
wherein a method for preparing the A) substance comprises: mixing the ginseng and the salvia, processing reflux extraction on the mixture with an aqueous solution of ethanol for obtaining extract and a first residue, collecting the extract, wherein the extract is the ethanol extract of the ginseng;
wherein a method for preparing the B) substance comprises: immersing the mint and the orange peel in water, processing distillation extraction for obtaining distillate and a second residue, wherein the distillate is the volatile oil of the mint and the orange peel;
wherein a method for preparing the C) substance comprises: mixing the peony, the poria, the licorice, the peach seed, the yam, the penthorum chienese, the first residue and the second residue, boiling, filtering and collecting filtrate, adding absolute ethanol and waiting for collecting supernatant, wherein the supernatant is the aqueous extract of the ginseng, the salvia, the mint, the orange peel, the peony, the poria, the licorice, the peach seed, the yam and the penthorum chienese;
wherein a method for preparing the D) substance comprises steps of: mixing the fruits and the vegetables, a bacteria liquid of *Lactobacillus acidophilus,* the bacteria liquid of the Bifidobactreium longum, the bacteria liquid of the Lactobacillus delbrueckiisubsp bulgaricus and the bacteria liquid of the Streptococcus thermophilus, fermenting for obtaining a fermentation product, wherein the fermentation product is the fermentation conversion solution of fruits and vegetables.

A mass ratio of the ginseng, the peony, the poria, the licorice, the orange peel, the yam, the mint, the peach seed, the salvia and the penthorum chienese is (1.5-2.5): (1.5-2.5): (1.9-2.9): (0.5-1.5): (0.8-1.7): (1.5-2.5): (0.8-1.7): (0.8-1.7): (1.5-2.5): (3.5-4.5);
preferably, the mass ratio of the ginseng, the peony, the poria, the licorice, the orange peel, the yam, the mint, the peach seed, the salvia and the penthorum chienese is (1.5, 2 or 2.5): (1.5, 2 or 2.5): (1.9, 2.4 or 2.9): (0.5, 1 or 1.5): (0.8, 1.2 or 1.7): (1.5, 2 or 2.5): (0.8, 1.2 or 1.7): (0.8, 1.2 or 1.7): (1.5, 2 or 2.5): (3.5, 4 or 4.5).

Preferably, in the methods for preparing the A) substance:
reflux extraction time is 2h-5h, the reflux extraction time is preferably 2h, 3h or 5h;
a concentration of the aqueous solution of ethanol is 70%-95% (according to volume fraction); the concentration is preferably 70%, 90% or 95%;
wherein in the methods for preparing the B) substance:
   immersion time is 1min-60min, the immersion time is preferably 1min, 30min or 60min;
   a distillation temperature is 90°C-100°C, the distillation temperature is preferably 90°C, 95°C or 100°C.
wherein in the method for preparing the C) substance:
   a boiling temperature is 90°C-100°C, the boiling temperature is preferably 90°C, 95°C or 100°C; a boiling time is 1h-2h, the boiling time is preferably 1h or 2h;
   a method for adding the absolute ethanol comprises adding the ethanol into the filtrate until a volume percentage of the ethanol in the filtrate is 40%;
   fermentation temperature is 18°C-37°C, the fermentation temperature is preferably 18°C, 23°C or 37°C, fermentation time is 10 days to 180 days, the fermentation time is preferably 10 days, 15 days or 180 days, a fermentation method comprises stirring while fermenting.

The method for preparing the A) substance further comprises steps of:
removing the ethanol from the extract, and then concentrating and drying for obtaining a dried product, wherein the dried product is the ethanol extract of the ginseng and the salvia;
the method for preparing the C) substance further comprises steps of:
   removing the ethanol from the supernatant after collecting the supernatant, filtering and collecting the filtrate, and then concentrating and drying for obtaining the dried product, wherein the dried product is the aqueous extract of the ginseng, the salvia, the mint, the orange peel, the peony, the poria, the licorice, the peach seed, the yam and the penthorum chienese;
   the method for preparing the D) substance further comprises a step of crushing the fruits and vegetables into 40-50 meshes size before the fermentation;
   after the fermentation, the method for preparing the D) substance further comprises steps of: filtering the fermentation product, collecting the filtrate, ultrafiltering, collecting ultrafiltrate, wherein the ultrafiltrate is the fermentation conversion solution of fruits and vegetables.

Preferably, ultrafiltration is filtering the filtrate with 100,000 molecular weight ultrafiltration membrane (an inputting pressure is 1.3kg, an outputting pressure is 0.5kg).

The fruits and vegetables are the mixture of the following 54 kinds of fruits and vegetables:
konjac, eggplant, asparagus, spinach, bean sprout, broccoli, cabbage, turnip, cucumber, pea, red pepper, celery, scallion, garlic, grape, grapefruit, watermelon, peach, orange, blueberry, orange, banana, lychee, bitter gourd, leek, pomegranate, dragon fruit, carrot, tomato, Chinese cabbage, celery, bell pepper, lettuce, pear, ginger, taro, bean, pumpkin, lotus root, cherry, kiwi, plum, strawberry, fig, kumquat, citrus, Southland pear, melon, cantaloupe, pawpaw, onion, mulberry, sugar beet and lemon.

Preferably, each fruit or vegetable is of the same weight.

In the method, 1L the mixture is prepared by: mixing (5-50)g the ethanol extract of the ginseng and the salvia, (5-50)g the volatile oil of the mint and the orange peel, (50-200)g the aqueous extract of the ginseng, the salvia, the mint, the orange peel, the peony, the poria, the licorice, the peach seed, the yam and the penthorum chienese and the fermentation conversion solution of the fruits and vegetables, adding the fermentation conversion solution of fruits and vegetables for making up a volume to 1L;
preferably, 1L the mixture is prepared by: mixing (5, 15 or 50)g the ethanol extract of the ginseng and the salvia, (5, 20 or 50)g the volatile oil of the mint and the orange peel, (50, 160 or 200)g the aqueous extract of the ginseng, the salvia, the mint, the orange peel, the peony, the poria, the licorice, the peach seed, the yam and the penthorum chienese and the fermentation conversion solution of the fruits and vegetables, adding the fermentation conversion solution of fruits and vegetables for making up a volume to 1L;
in the method for preparing the A) substance:
a ratio of the aqueous solution of the ethanol, the ginseng and the salvia is (1-10)ml: 0.5g: 0.5g;
preferably, the ratio of the aqueous solution of the ginseng and the salvia is (1, 5 or 10)ml: 0.5g: 0.5g;
in the method for preparing the B) substance:
   the ratio of the mint, the orange peel and the water is (1-10)g: (1-10)g: (1-10)ml;
   preferably, the ratio of the mint, the orange peel and the water is (1, 5 or 10)g: (1, 5 or 10)g: (1, 5 or 10)ml;
in the method for preparing the C) substance:
   the ratio of the peony, the poria, the licorice, the peach seed, the yam, the penthorum chienese and the water is (1-10)g: (1-10)g: (1-10)g: (1-10)g: (1-10)g: (1-10)g: (10-100)ml;
   preferably, the ratio of the peony, the poria, the licorice, the peach seed, the yam, the penthorum chienese and the water is (1, 2 or 10)g: (1, 2.4 or 10)g: (1, 5 or 10)g: (1, 1.2 or 10)g: (1, 2 or 10)g: (1, 4 or 10)g: (50, 60 or 100)ml;
   wherein the penthorum chienese is a dried aboveground part of penthorum chinense pursh of the family Saxifragaceae of the genus Penthorum.

In the method for preparing the D) substance:
the ratio of the bacteria liquid of the *Lactobacillus acidophilus,* the bacteria liquid of the *Bifidobactreium longum,* the bacteria liquid of the *Lactobacillus delbrueckiisubsp bulgaricus,* the bacteria liquid of the *Streptococcus thermophilus,* the fermentation conversion solution of the fruits and vegetables and water is (2000-8000)ml: (2000-8000)ml: (2000-8000)ml: (2000-8000)ml: (1000-1500)kg: (1000-1500)kg;
preferably, the ratio of the bacteria liquid of the *Lactobacillus acidophilus,* the bacteria liquid of the *Bifidobactreium longum,* the bacteria liquid of the *Lactobacillus delbrueckiisubsp bulgaricus,* the bacteria liquid of the *Streptococcus thermophilus,* the fermentation conversion solution of the fruits and vegetables and the water is (2000, 5000 or 8000)ml: (2000, 5000 or 8000)ml: (2000, 5000 or 8000)ml: (2000, 5000 or 8000)ml: (1000, 1200 or 1500)kg: (1000, 1200 or 1500)kg.

A method for preparing the bacteria liquid of the *Lactobacillus acidophilus* comprises: fermenting and growing *Lactobacillus acidophilus* for obtaining the fermentation production, wherein the fermentation production is the bacteria liquid of the *Lactobacillus acidophilus;* wherein the fermentation temperature is 20°C-41°C, the fermentation temperature is preferably 20°C, 37°C or 41°C, the fermentation time is 15h-36h, the fermentation time is preferably 15h, 16h or 36h;
a method for preparing the bacteria liquid of the *Bifidobactreium longum* comprises: fermenting and growing *Bifidobactreium longum* for obtaining the fermentation production, wherein the fermentation production is the bacteria liquid of the *Bifidobactreium longum;* wherein the fermentation temperature is 20°C-41°C, the fermentation temperature is preferably 20°C, 37°C or 41°C, the fermentation time is 15h-36h, the fermentation time is preferably 15h, 16h or 36h;
a method for preparing the bacteria liquid of the *Lactobacillus delbrueckiisubsp bulgaricus* comprises: fermenting and growing *Lactobacillus delbrueckiisubsp bulgaricus* for obtaining the fermentation production, wherein the fermentation production is the bacteria liquid of the *Lactobacillus delbrueckiisubsp bulgaricus;* wherein the fermentation temperature is 20°C-41°C, the fermentation temperature is preferably 20°C, 37°C or 41°C, the fermentation time is 15h-36h, the fermentation time is preferably 15h, 16h or 36h;
a method for preparing the bacteria liquid of the *Streptococcus thermophilus* comprises: fermenting and growing *Streptococcus thermophilus* for obtaining the fermentation production, wherein the fermentation production is the bacteria liquid of the *Streptococcus thermophilus;* wherein the fermentation temperature is 20°C-41°C, the fermentation temperature is preferably 20°C, 37°C or 41°C, the fermentation time is 15h-36h, the fermentation time is preferably 15h, 16h or 36h.

The *Lactobacillus acidophilus* is the *Lactobacillus acidophilus* CGMCC (China General Microbiological Culture Collection) 1.1854, the *Bifidobactreium longum* is the *Bifidobactreium longum* CGMCC 1.2186, the *Lactobacillus delbrueckiisubsp bulgaricus* is the *Lactobacillus delbrueckiisubsp bulgaricus* CGMCC 1.1480, the *Streptococcus thermophilus* is the *Streptococcus thermophilus* CGMCC 1.2471.

A growth medium prescription for fermental cultivation: mixing 10g peptone, 10g beef extract, 5g yeast extract, 20g glucose, 1g Tween-80, 2g K₂HPO₄, 1g Tween-80, 5g NaAC, 2g triammonium citrate, 0.2g MgSO₄, 0.05g MnSO₄ and the water, making up to 1L by the water for obtaining the growth medium. The pharmaceutical composition prepared by the above methods for treating the melasma is also within protection scope of the present invention.

The pharmaceutical composition for treating the melasma prepared by the method is within the scope of the present invention.

Experiments of the present invention illustrates that extracting time for obtaining the ethanol extract of the ginseng and the salvia, the volatile oil of the mint and the orange peel, the aqueous extract of the ginseng, the salvia, the mint, the orange peel, the peony, the poria, the licorice, the peach seed, the yam and the penthorum chienese and the fermentation conversion solution of the fruits and vegetables is short.

These and other objectives, features, and advantages of the present invention will become apparent from the following detailed description, the accompanying drawings, and the appended claims.

### Detailed Description of the Preferred Embodiment

Experimental methods used in the following preferred embodiments are conventional methods except for special instructions.

Ingredients and agents used in the following preferred embodiments are commercially available except for special instructions.

Preferred embodiment 1: obtaining ferment composition of ginseng for treating melasma
Method I:
wherein the active ingredients of the ferment composition of the ginseng are:
200g the ginseng, 200g peony, 240g poria, 100g licorice, 120g orange peel, 200g yam, 120g mint, 120g peach seed, 200g salvia and 400g penthorum chienese;
a) preparing ethanol extract of the ginseng and the salvia:
1. preparing the ingredient: wherein the ginseng and the salvia purchased from Jilin Zixin Pharmaceutical Industrial Co., Ltd are in line with the Pharmacopoeia of the People's Republic of China; quality is strictly controlled before feeding according to corporate standards;
2. weighting the ginseng and the salvia according to a prescription, washing with water and drying the ginseng and the salvia, processing reflux extraction twice with a 10-flod (v/w) 90% aqueous solution of ethanol, reflux extraction time for once is 3h, filtrating extract, combining filtrate, vacuum recovering the ethanol; wherein residues are ginseng residues and salvia residues; and
3. concentrating the filtrate to a relative density of 1.10-1.15 (60°C, 0.07MPa), spray-drying the filtrate (an inputting temperature is 170°C, an outputting temperature is 70°C) for obtaining spray-dried powder, wherein the powder is the ethanol extract of the ginseng and the salvia;

b) preparing volatile oil of the mint and the orange peel:
1. preparing the ingredient: wherein the mint and the orange peel purchased from Jilin Zixin Pharmaceutical Industrial Co., Ltd are in line with the Pharmacopoeia of the People's Republic of China; the quality is strictly controlled before feeding according to the corporate standards; and
2. weighting the mint and the orange peel according to the prescription, washing with the water and drying the mint and the orange peel, wherein immersion time is 30min, the water should fully immerse the mint and the orange peel; adding 5-flod the water, distilling at 100°C, collecting distillate with another container with a ratio of 5: 1 (wherein a volume of the distillate is 1/5 of the volume of the water for completely collecting the distillate), wherein the residues are mint residues and orange peel residues; the ration of the mint, the orange peel and the water is 5g: 5g: 5ml;

c) preparing aqueous extract of the ginseng, the salvia, the mint, the orange peel, the peony, the poria, the licorice, the peach seed, the yam and the penthorum chienese:
1. preparing the ingredient: wherein the peony, the poria, the licorice, the peach seed, the yam and the penthorum chienese purchased from Jilin Zixin Pharmaceutical Industrial Co., Ltd are in line with the Pharmacopoeia of the People's Republic of China; the quality is strictly controlled before feeding according to the corporate standards;
2. weighting the peony, the poria, the licorice, the peach seed, the yam and the penthorum chienese according to the prescription, washing with the water and drying the peony, the poria, the licorice, the peach seed, the yam and the penthorum chienese; and
3. mixing the ginseng residues, the salvia residues, the mint residues, the orange peel residues, the peony, the poria, the licorice, the peach seed, the yam and the penthorum chienese, boiling at 100°C twice, wherein the water is respectively 6 times and 5 times of the mixture, boiling time is respectively 2h and 1h; combining twice and concentrating the mixture until a relative density is 1.12-1.20 (by vacuum concentrating), adding ethanol and stirring until a volume percentage of the ethanol is 40%, waiting for 24h, collecting supernatant and filtering after the ethanol is completely recovered; concentrating the filtrate to the relative density of 1.10-1.15 (60°C, 0.07MPa), spray-drying the filtrate (the inputting temperature is 170°C, the outputting temperature is 70°C) for obtaining the spray-dried powder, wherein the powder is the aqueous extract of the ginseng, the salvia, the mint, the orange peel, the peony, the poria, the licorice, the peach seed, the yam and the penthorum chienese, the ratio of the peony, the poria, the licorice, the peach seed, the yam, the penthorum chienese and the water is 2g: 2.4g: 1g: 1.2g: 2g: 4g: 60ml;

d) preparing a fermentation conversion solution of fruits and vegetables:
1. selecting the fruits and vegetables materials;

**Table 1: fruits and vegetables selected**

| Ingredient | nutritional content | Ingredient | nutritional content |
|---|---|---|---|
| konjac | vitamin B1, B2, citric acid, fermentation conversion matter | carrot | vitamin A, carotene, fermentation conversion matter |
| eggplant | vitamin A, B1, B2, C, fermentation conversion matter | tomato | vitamin A, carotene, citric acid, fermentation conversion matter |
| asparagus | vitamin B1, B2, citric acid, fermentation conversion matter | Chinese cabbage | vitamin, mineral substance, fermentation conversion matter |
| spinach | vitamin A, C, calcium, iron, fermentation conversion matter | celery | vitamin, mineral substance, cellulose, fermentation conversion matter |
| bean sprout | vitamin, saponin, amino acid, fermentation conversion matter | bell pepper | vitamin C, mineral substance, fermentation conversion matter |
| broccoli | vitamin B1, B2, citric acid, fermentation conversion matter | lettuce | vitamin A, mineral substance, fermentation conversion matter |
| cabbage | vitamin B1, B2, citric acid, fermentation conversion matter | pear | fructose, mineral substance, fermentation conversion matter |
| turnip | vitamin B1, B2, citric acid, fermentation conversion matter | ginger | vitamin, mineral substance, fermentation conversion matter |
| cucumber | vitamin B1, B2, citric acid, fermentation conversion matter | taro | vitamin B1, B2, C, mineral substance, fermentation conversion matter |
| pea | vitamin B1, B2, citric acid, fermentation conversion matter | bean | vitamin B1, B2, citric acid, carotene, fermentation conversion matter |
| red pepper | vitamin B1, B2, citric acid, fermentation conversion matter | pumpkin | carotene, mineral substance,fermentation conversion matter |
| celery | vitamin B1, B2, citric acid, fermentation conversion matter | lotus root | iron, tannin, fermentation conversion matter |
| scallion | vitamin B1, B2, citric acid, fermentation conversion matter | cherry | mineral substance, fermentation conversion matter |
| garlic | vitamin B1, B2, citric acid, fermentation conversion matter | kiwi | vitamin C, fermentation conversion matter |
| grape | vitamin B1, B2, citric acid, fermentation conversion matter | plum | organic acid, vitamin, fermentation conversion matter |
| grapefruit | vitamin B1, B2, citric acid, fermentation conversion matter | strawberry | vitamin C, mineral substance, ellagic acid, fermentation conversion matter |
| watermelon | vitamin B1, B2, citric acid, fermentation conversion matter | fig | digest fermentation conversion matter, vitamin, mineral substance |
| peach | vitamin B1, B2, citric acid, fermentation conversion matter | kumquat | vitamin B1, B2, C, fermentation conversion matter |
| orange | vitamin B1, B2, C, fermentation conversion matter | citrus | vitamin C, citric acid, fermentation conversion matter |
| blueberry | vitamin B1, B2, citric acid, fermentation conversion matter | Sounthland pear | vitamin B1, B2, C, citric acid, fermentation conversion matter |
| orange | vitamin B1, B2, citric acid, fermentation conversion matter | melon | vitamin B1, B2, citric acid, fermentation conversion matter |
| banana | vitamin B1, B2, citric acid, fermentation conversion matter | cantaloupe | fructose, potassium, vitamin A, fermentation conversion matter |
| lychee | vitamin B1, B2, citric acid, fermentation conversion matter | pawpaw | vitamin B, C, E, citric acid, carotene, fermentation conversion matter |
| bitter gourd | vitamin B1, B2, citric acid, fermentation conversion matter | onion | vitamin B, C, carotene, fermentation conversion matter |
| leek | vitamin B1, B2, citric acid, fermentation conversion matter | mulberry | vitamin B1, B2, citric acid, fermentation conversion matter |
| pomegranate | vitamin B1, B2, citric acid, fermentation conversion matter | sugar beet | betaine, fermentation conversion matter |
| dragon fruit | vitamin B1, B2, citric acid, fermentation conversion matter | lemon | citric acid, fermentation conversion matter |

2. fermenting and converting the fruits and vegetables, comprising steps of:
2-a) selecting and purchasing cultures;
   wherein probiotics culture is purchased from Institute of Microbiology Chinese Academy of Sciences, probiotics is respectively *Lactobacillus acidophilus* CGMCC 1.1854, *Bifidobactreium longum* CGMCC 1.2186, *Lactobacillus delbrueckiisubsp bulgaricus* CGMCC 1.1480 and *Streptococcus thermophilus* CGMCC 1.2471, the end of all the cultures are preserved as a starter community in sand tubes;
2-b) preparing main seeds;
   wherein preparation of the probiotic main seed comparses steps of (the main seed should be reproduced for no more than ten generations, and the main seed in the preparation is reproduced for four generations):
   2-b1) respectively taking a 1/10 sand tube amount of the *Lactobacillus acidophilus* CGMCC 1.1854, the *Bifidobactreium longum* CGMCC 1.2186, the *Lactobacillus delbrueckiisubsp bulgaricus* CGMCC 1.1480 and the *Streptococcus thermophilus* CGMCC 1.2471 with a sterile stainless steel spoon, freezing and storing the rest, respectively connecting the sand tubes to 50ml (250 flask) MRS liquid growth medium (the growth medium comprises 10g/l peptone, 10g/l beef extract, 5g/l yeast extract, 20g/l glucose, 1g/l Tween-80, 2g/l K₂HPO₄, 1g/l Tween-80, 5g/l NaAC, 2g/l triammonium citrate, 0.2g/l MgS0₄ and 0.05g/l MnS0₄, the growth medium is sterilized at 121°C for 20min), growing at 37°C with a 100rpm shaker for 16h;
   2-b2) picking a loop of each colony, respectively streaking the colony loop on MRS solid growth medium (the growth medium comprises 10g/l peptone, 10g/l beef extract, 5g/l yeast extract, 20g/l glucose, 1g/l Tween-80, 2g/l K₂HPO₄, 1g/l Tween-80, 5g/l NaAC, 2g/l triammonium citrate, 0.2g/l MgS0₄, 0.05g/l MnS0₄ and 1.5% agar, the growth medium is sterilized at 121°C for 20min), growing at 37°C with an incubator for 16h;
   2-b3) picking the best-growing colony form each the loop, respectively connecting the colony to the 50 ml MRS liquid growth medium, growing at 30°C with the 180rpm shaker for 16h; and
   2-b4) respectively connecting to the 500ml MRS liquid growth medium, growing at 37°C with the 100rpm shaker for 16h, adding glycerol until the glycerol takes a proportion of 20%, shaking up, respectively dividing into 1ml and inputting into a freezing tube as the main seed of the probiotic *Lactobacillus acidophilus,* the *Bifidobactreium longum,* the *Lactobacillus delbrueckiisubsp bulgaricus* or the *Streptococcus thermophilus,* preservating at -40°C;

3. preparing working seeds:
wherein preparation of probiotic working seed comparses steps of (the working seed should be reproduced for no more than five generations, and the working seed in the preparation is reproduced for four generations):
   3-a) respectively taking the main seeds of the *Lactobacillus acidophilus* CGMCC 1.1854, the *Bifidobactreium longum* CGMCC 1.2186, the *Lactobacillus delbrueckiisubsp bulgaricus* CGMCC 1.1480 and the *Streptococcus thermophilus* CGMCC 1.2471 with a sterile inoculating loop, respectively streaking to the MRS solid growth medium, growing at 37°C with the incubator for 16h;
   3-b) picking the best-growing colony form each the loop, respectively connecting the colony to the 50 ml MRS liquid growth medium, growing at 37°C with the 100rpm shaker for 16h;
   3-c) respectively connecting to the 500ml MRS liquid growth medium, growing at 37°C with the 100rpm shaker for 16h; and
   3-d) respectively connecting to the 5000ml MRS liquid growth medium, growing at 37°C with the 100rpm shaker for 16h, obtaining the bacteria liquid of the the probiotic *Lactobacillus acidophilus,* the *Bifidobactreium longum,* the *Lactobacillus delbrueckiisubsp bulgaricus* and the *Streptococcus thermophilus;* wherein the above bacteria liquid is all fermentation products in a fermentation container;

4. processing the fruits and vegetables:
4-a) weighing the fruits and vegetables;
4-b) washing, draining and weighing; and
4-c) crushing into 40-50 meshes size, putting into a fermentation tank;
wherein for the 3-ton fermentation tank (an effective volume = 3tons × 0.8 = 2.4tons), an actual feeding amount is controlled at 2400kg; the fruits and vegetables: water is 1: 1, feeding 1200kg the fruits and vegetables, then adding 1200kg the water;
5. fermenting and converting a rapid enzymic hydrolysate of the fruits and vegetables:
5-a) respectively adding 5000ml the bacteria liquid of the the *Lactobacillus acidophilus* CGMCC 1.1854, the *Bifidobactreium longum* CGMCC 1.2186, the *Lactobacillus delbrueckiisubsp bulgaricus* CGMCC 1.1480 and the *Streptococcus thermophilus* CGMCC 1.2471, controlling a fermentation temperature at 23°C, stirring for 15 days;
5-b) filtering with 200 meshes size a filter cloth, abandoning pomace, obtaining the filtrate; and
5-c) ultrafiltrating with 100,000 molecular weight ultrafiltration membrane (an inputting pressure is 1.3kg, an outputting pressure is 0.5kg), obtaining clear liquid, sealing and preserving at 4°C, wherein the clear liquid is the fermentation conversion solution of the fruits and vegetables; and

6. testing:
wherein the main products of the fermentation conversion solution of the fruits and vegetables is mainly lactic acid and acetic acid, therefore, an acidity is identified as a characteristic component;
the acidity of the fermentation conversion solution of the fruits and vegetables: referring to an amount of a 0.1N NaOH solution used in milliliter for titrating 100 ml the fermentation conversion solution of fruits and vegetables, wherein 10ml samples are often used in a test;
a method for testing comprises steps of: taking 10ml the fermentation conversion solution of the fruits and vegetables, adding 20ml the water and 0.5ml 0.5% phenolphthalein indicator, titrating with the 0.1N NaOH until the liquid is reddish and doesn't fade within 30sec; calculating: the acidity = the amount of the 0.1N NaOH solution used × 10;
result: the acidity of the fermentation conversion solution of the fruits and vegetables is 42; and

e) preparing composition:
mixing the above extract and the fermentation conversion solution of the fruits and vegetables according to the following formulation:
   mixing 15g the ethanol extract of the ginseng and the salvia, 20g the volatile oil of the mint and the orange peel, 160g the aqueous extract of the ginseng, the salvia, the mint, the orange peel, the peony, the poria, the licorice, the peach seed, the yam and the penthorum chienese and the fermentation conversion solution of the fruits and vegetables, adding the fermentation conversion solution of the fruits and vegetables for making up the volume to 10,000ml, wherein the mixture is the composition, dividing into 1000 bottles with 10ml/bottle for future usage.

Method II:
wherein the active ingredients of the ferment composition of ginseng are: 150g the ginseng, 150g the peony, 190g the poria, 50g the licorice, 80g the orange peel, 150g the yam, 80g the mint, 80g the peach seed, 150g the salvia and 350g the penthorum chienese;
   a) preparing the ethanol extract of the ginseng:
      the extracting method is basically the same as the method in the method I, deference is that the ginseng is reflux-extracted by the 1-flod (v/w) 70% aqueous solution of the ethanol and the reflux extraction time is 2h for once;
   b) preparing the volatile oil of the mint and the orange peel:
      the extracting method is basically the same as the method in the method I, the deference is that the ratio of the mint, the orange peel and the water is 1g: 1g: 1 ml; the immersion time is 1min; the distillation temperature is 90°C;
   c) preparing aqueous extract of the ginseng, the salvia, the mint, the orange peel, the peony, the poria, the licorice, the peach seed, the yam and the penthorum chienese:
      the extracting method is basically the same as the method in the method I, the deference is that the ratio of the peony, the poria, the licorice, the peach seed, the yam, the penthorum chienese and the water is 1g: 1g: 5g: 1g: 1g: 1g: 50ml; the boiling time is 90°C;
   d) preparing the fermentation conversion solution of the fruits and vegetables:
      the extracting method is basically the same as the method in the method I, the deference is respectively adding 2000ml the bacteria liquid of the the *Lactobacillus acidophilus* CGMCC 1.1854, the *Bifidobactreium longum* CGMCC 1.2186, the *Lactobacillus delbrueckiisubsp bulgaricus* CGMCC 1.1480 and the *Streptococcus thermophilus* CGMCC 1.2471, adding 1000kg the fruits and vegetables and adding 1000kg the water;
      wherein the fermentation time is 18°C, the fermentation time is 10 days;
      wherein in the preparation of the bacteria liquid of the *Lactobacillus acidophilus,* the *Bifidobactreium longum,* the *Lactobacillus delbrueckiisubsp bulgaricus* and the *Streptococcus thermophilus,* the fermentation time is 20°C, the fermentation time is 15h;
      wherein the testing method is the same as the method in the method I, the result has no significant difference; and
   e) preparing the composition:
      the preparing method is basically the same as the method in the method I, the deference is mixing 5g the ethanol extract of the ginseng, 5g volatile oil of the mint and the orange peel, 50g aqueous extract of the ginseng, the salvia, the mint, the orange peel, the peony, the poria, the licorice, the peach seed, the yam and the penthorum chienese and the fermentation conversion solution of the fruits and vegetables; adding the fermentation conversion solution of the fruits and vegetables for making up the volume to 10,000ml, wherein the mixture is the composition.

Method III,
wherein the active ingredients of the ferment composition of ginseng are: 250g the ginseng, 250g the peony, 290g the poria, 150g the licorice, 170g the orange peel, 250g the yam, 170g the mint, 170g the peach seed, 250g the salvia and 450g the penthorum chienese;;
a) preparing the ethanol extract of the ginseng:
   the extracting method is basically the same as the method in the method I, deference is that the ginseng is reflux-extracted by the 5-flod (v/w) 95% aqueous solution of the ethanol;
b) preparing the volatile oil of the mint and the orange peel:
   the extracting method is basically the same as the method in the method I, the deference is that the ratio of the mint, the orange peel and the water is 10g: 10g: 10ml; the immersion time is 60min; the distillation temperature is 95°C;
c) preparing aqueous extract of the ginseng, the salvia, the mint, the orange peel, the peony, the poria, the licorice, the peach seed, the yam and the penthorum chienese:
   the extracting method is basically the same as the method in the method I, the deference is that the ratio of the peony, the poria, the licorice, the peach seed, the yam, the penthorum chienese and the water is 10g: 10g: 10g: 10g: 10g: 10g: 100ml; the boiling time is 95°C;
d) preparing the fermentation conversion solution of the fruits and vegetables:
   the extracting method is basically the same as the method in the method I, the deference is respectively adding 8000ml the bacteria liquid of the the *Lactobacillus acidophilus* CGMCC 1.1854, the *Bifidobactreium longum* CGMCC 1.2186, the *Lactobacillus delbrueckiisubsp bulgaricus* CGMCC 1.1480 and the *Streptococcus thermophilus* CGMCC 1.2471, adding 1500kg the fruits and vegetables and adding 1500kg the water;
   wherein the fermentation time is 37°C, the fermentation time is 180 days;
   wherein in the preparation of the bacteria liquid of the the *Lactobacillus acidophilus,* the *Bifidobactreium longum,* the *Lactobacillus delbrueckiisubsp bulgaricus* and the *Streptococcus thermophilus,* the fermentation time is 41°C, the fermentation time is 36h;
   wherein the testing method is the same as the method in the method I, the result has no significant difference; and
i) preparing the composition:
   the preparing method is basically the same as the method in the method I, the deference is mixing 50g the ethanol extract of the ginseng, 50g volatile oil of the mint and the orange peel, 200g aqueous extract of the ginseng, the salvia, the mint, the orange peel, the peony, the poria, the licorice, the peach seed, the yam and the penthorum chienese and the fermentation conversion solution of the fruits and vegetables; adding the fermentation conversion solution of the fruits and vegetables for making up the volume to 10,000ml, wherein the mixture is the composition.

Preferred embodiment 2: preparing ginseng oral liquid for treating the melasma

Conventional production processes of oral liquid are utilized in the present invention: mixing → filtering → instantaneously sterilizing →canning → capping → leak testing → clarity checking → packaging → testing → storing, wherein the processes before the leak testing are provided in 100,000 class clean area. A production process design and equipment conditions are in line with GMP (Good Manufacturing Practice) requirements of China.

Specifically, a production method comprises steps of:
1. mixing the composition in the method I of the preferred embodiment 1 with a sweetener (specifically, the sweetener is white sugar purchased from Changchun Eurasia department store, w / v), then putting into a make-up tank, mixing for 30min for shaking up, instantaneously sterilizing by high heat, wherein the product is the ginseng oral liquid for treating the melasma;
2. canning the oral liquid: wherein the oral liquid is canned 10ml per bottle by filling machines, volumes of the canned oral liquid should be detected during canning in such a manner that difference between the volumes is controlled within the range;
3. capping: wherein the bottle is capped by capping machines, the unqualified products are screened out;
4. leak testing: wherein the capped bottles are tested by a vacuum leak detector;
5. inner packaging: wherein cardboard boxes are utilized, and each the box contains 10 bottles;
6. outer packaging: wherein corrugated boxes are utilized;
7. testing: wherein the products are tested according to the corporate standards;
8. storing: wherein the products are stored in warehouses after passing the tests.

Preferred embodiment 3: study on anti-melasma effects of the ginseng oral liquid for treating the melasma
1. experiment materials and methods:
1.1. testing medicine:
the ginseng oral liquid for treating the melasma is prepared in the preferred embodiment 2, vitamin C pills are purchased from Sichuan Furui Pharmaceutical Industrial Co., Ltd;

1.2. animals:
Kunming female mice weighting 28±2g, provided by the Experimental Animal Center of Jilin University;

1.3. reagents:
progesterone injection: wherein the progesterone injection is 10mg/ml and a serial number is 060314, the progesterone injection is purchased from Zhejiang Xianju Pharmaceutical Industrial Co., Ltd; superoxide dismutase (SOD): wherein the serial number is 20060803; malondialdehyde (MDA): wherein the serial number is 20060802; nitrogen monoxide (NO): wherein the serial number is 20060728; coomassie brilliant blue: wherein the serial number is 20060731; reagent kits are all provided by Nanjing Jiancheng Bioengineering Institute; a mouse anti-human McAb (monoclonal antibody) HMB45 (melanoma) ready kit MAB-0098 is produced by Fujian Maixin Pharmaceutical Industrial Co., Ltd; a concentration DAB (diaminobenzidine) kit is produced by Beijing Zhongshan Jinqiao Pharmaceutical Industrial Co., Ltd; and

1.4. the experimental methods:
randomly dividing 84 the mice into six groups; injecting the mice of a normal group with sterilized water with a dosage of 2ml/kg; injecting the mice of the other groups with the progesterone in muscles of rear legs, wherein the left rear leg and the right rear leg are injected alternatively, the dosage is 20mg/kg (equals to eight times of a clinical dosage of the human); injecting for 6d a week and lasting for 30d; feeding the medicine or distilled water with the dosage of 0.2ml/10g while modeling, wherein the medicine or the distilled water are fed once a day for 30d; wherein the dosages of a low group, a medium group and a high group are 12, 24 and 36g/kg of the ginseng oral liquid (equals to five, ten, fifteen times of the clinical dosage); the dosage of the vitamin C is 0.1g/kg (equals to ten times of a clinical dosage of the human); sampling 1ml blood from orbits of the mice of each the group 1h after the last feeding, putting in a capped plastic tube comprising dried heparin, detecting a viscosity the whole blood; executing all the mice and removing hairs on backs, collecting livers and skins, washing with precooled saline, drying with filter paper; taking 0.5g skin and 0.5g liver, adding into the cold saline with the ratio of 1: 9 (w/v), processing bath for obtaining 10% tissue homogenate, separating by a 2500r/min centrifugal separator, taking the supernatant and detecting relative parameters;

1.4.1. effects on the viscosity of the whole blood of the mice:
detecting the viscosity of the whole blood by a blood viscosity analyzer, wherein it is illustrated by table 2 that when compared with the normal control group, the blood viscosities of the mice of the modeling control group and the vitamin C group with different shear rates are obviously increased; when compared with the modeling control group, the blood viscosities of the high group with the ginseng oral liquid for treating the melasma are obviously decreased, which means that the ferment composition of the ginseng can effectively improve a rheology of the blood of the mouse with the melasma as well as decrease the blood viscosity;

**Table 2: effects of ferment composition of ginseng on viscosity of whole blood of mice (x±s)**

| | | | blood viscosity with different shear rates (mPas) | | | |
|---|---|---|---|---|---|---|
| group | dosage (ml) | number | 1/s | 3/3 | 30/s | 100/s |
| normal control | 0 | 10 | 22.74±3.08 | 16.58±2.48 | 8.20±1.90 | 3.66±0.84* |
| modeling control | 0 | 11 | 30.93±5.70 | 21.81±4.38 | 11.39±2.80 | 4.83±1.73 |
| high | 30 | 9 | 22.26±3.12* | 18.13±2.85* | 9.33±2.30* | 3.85±1.03 |
| medium | 20 | 10 | 24.12±4.26 | 19.11±3.34 | 10.00±2.33 | 4.00±1.17 |
| low | 10 | 9 | 27.84±2.76 | 18.96±4.04 | 10.24±2.08 | 4.27±0.78 |
| vitamin C | 0.1 | 10 | 29.22±2.18 | 28.85±4.35 | 11.22±1.75 | 4.60±1.36 |

| | | | | | | |
|---|---|---|---|---|---|---|
| Note: comparison with the modeling groups, *P < 0.05, **P < 0.01 (same as below); | | | | | | |

1.4.2. detecting SOD, MDA and NO parameters:
detecting with a 722s spectrophotometer according to instructions of the reagent kits and operation requirements; wherein effects on the SOD, the MDA and the NO contents in mice livers are illustrated in table 3, when compared with the normal control group, SOD activities in the livers of the mice of the modeling control group are decreased, MDA and NO contents are increased, and great difference exists in the MDA content; when compared with the modeling control group, the SOD activities of the high group with the ginseng oral liquid and the vitamin C group are obviously increased, and the MDA contents of the high group are obviously decreased;

**Table 3: effects of ferment composition of ginseng on SOD, MDA and NO contents in mice livers (x±s)**

| group | dosage (ml) | number | SOD | MDA | NO |
|---|---|---|---|---|---|
| normal control | 0 | 10 | 37.47±18.05 | 3.25±1.61 | 1.24±0.94 |
| modeling control | 0 | 11 | 32.48±8.62 | 5.53±2.55 | 1.98±1.17 |
| high | 30 | 9 | 52.15±14.82* | 3.78±1.51* | 1.66±0.60 |
| medium | 20 | 10 | 42.76±16.20 | 4.5±2.04 | 1.60±1.00 |
| low | 10 | 9 | 36.15±10.77 | 4.62±1.21 | 1.98±0.83 |
| vitamin C | 0.1 | 10 | 45.62±16.04* | 4.58±1.26 | 1.90±0.93 |

wherein the effects on the SOD, the MDA and the NO contents in mice skins are illustrated in table 4, when compared with the normal control group, the SOD activities in the skins of the mice of the modeling control group are decreased, the MDA and the NO contents are increased, and great difference exists in the MDA content; when compared with the modeling control group, the SOD activities of the high group with the ginseng oral liquid and the medium group are obviously increased, and the MDA contents of the high group are obviously decreased; when compared with the modeling control group, the MDA contents of the vitamin group are obviously decreased;

**Table 4: effects of ferment composition of ginseng on SOD, MDA and NO contents in mice skins (x±s)**

| group | dosage (ml) | number | SOD | MDA | NO |
|---|---|---|---|---|---|
| normal control | 0 | 10 | 15.94±3.73 | 3.50±1.08 | 1.17±0.67 |
| modeling control | 0 | 10 | 14.85±5.07 | 5.40±1.06 | 1.73±0.79 |
| high | 30 | 10 | 23.91±9.17* | 3.54±1.24* | 1.25±0.45 |
| medium | 20 | 10 | 21.80±6.24* | 3.83±1.21* | 1.72±0.59 |
| low | 10 | 10 | 18.87±6.30 | 4.58±1.13 | 1.72±0.92 |
| vitamin C | 0.1 | 10 | 19.95±5.33 | 4.20±1.34* | 1.71±0.52 |

1.4.3. observing melanin cells in the skins according to pathomorphology:

Stripping a block of the back skin of the mouse, removing the hairs, fixing with 10% neutral formalin, embedding in conventional paraffin, cutting into 5 µ m thick sections continuously, and staining with HE (hematoxylin-eosinstaining); marking the melanin by immunohistochemistry method, utilizing biotin method for obtaining streptomyces, selecting the mouse anti-human McAb HMB45 (melanoma) as a first antibody, coloring by the DAB, and coloring again with hematoxylin; processing negative control with PBS (phosphate buffered saline) instead of the first anti-body; processing computer image analysis on melanin-positive target: randomly selecting skins form eight mice of each group, obtaining a tissue section from each the skin, observing five nonoverlapping fields of view, and finding the positive targets; quantitatively analyzing by a MIAS2000 computer image analysis system for obtaining 5 views of each the tissue section and average size, average optical density and integrated optical density of the melanin-positive target of the eight animals of each the group;
wherein observing the melanin cells stained by the HE in the skins according to the pathomorphology: the melanin cells can not be found under a light microscope; observing the HMB45 mark: a small number of the melanin cells can be found in hair follicles and sebaceous gland basal cells of the mice of the normal control group, the melanin cells cluster in the hair follicles and the sebaceous gland basal cells of the mice of the modeling control group, and can also be found in epidermis basal cells and prickle cell layer, the melanin cells only scatter in the basal cells of basal layer and the hair follicles of the mice of the four groups with the medicine;
effects on the melanin cells in the mice skins are illustrated in table 5, wherein when compared with the normal control group, the average size, the average optical density and the integrated optical density of the mouse of the modeling control group are significantly increased; when compared with the modeling control group, the average optical density and the integrated optical density of the high and medium groups are significantly decreased;

**Table 5: effects of ferment composition of ginseng on melanin cells in skins of mice (x±s)**

| group | dosage (ml) | number | average size of positive target | average optical density | integrated optical density |
|---|---|---|---|---|---|
| normal control | 0 | 10 | 2893.47±923.51 | 0.24±0.08 | 851.52±314.35 |
| modeling control | 0 | 11 | 4116.55±1175.89 | 0.39±0.11 | 1421.52±532.47 |
| high | 30 | 9 | 3480.95±1206.12 | 0.26±0.09* | 883.15±345.00* |
| medium | 20 | 10 | 3620.40±1204.21 | 0.28±0.10* | 985.27±321.91* |
| low | 10 | 9 | 3910.85±1365.11 | 0.34±0.12 | 1226.52±453.44 |
| vitamin C | 0.1 | 10 | 3720.56±1191.13 | 0.33±0.14 | 1152.52±344.09 |

The melasma is a multi-factor, multi-system, multi-link pigmented skin problem, pathogenesis thereof is complex, and a real cause is not yet clear. As a pigmented skin problem, too much melanin generated is a basic reason, synthesis of the melanin by the melanin cells is a very complex process, and is affected by a variety of factors. Hemodynamic parameters of patients with the melasma are abnormal, therefore, changes of the hemodynamic parameters are considered to have a close relationship with the melasma.

Detection of the hemodynamic parameters provides an experimental basis for treating the disease.

In recent years, many studies have indicated that oxygen free radicals are responsible for formation of the melanin and pigmentation in the skin. The MDA can lead to cross-link of protein molecules for forming a fluorescent chromophore (pigment). In addition to the effect of removing the free radicals, the SOD can also inhibit the activity of tyrosinase and reduce the pigmentation. A large amount of data indicated that the activities of the SOD and catalase (CAT) in the blood of the patient with the melasma are obviously decreased, and the contents of LP0 and the MDA are obviously increased. The above studies illustrate that imbalance between oxidation and antioxidant may be an important factor for the melasma.

In many signal-medicated methods for affecting function of the melanin cells, the NO is considered to be an extremely important signal molecule with an important role. The NO can stimulate the activity of the tyrosinase, and improve the amount of the new synthesized melanin.

Experiment results according to the present invention indicated that the ginseng oral liquid for treating the melasma has the effects of promoting blood circulation and removing blood stasis, and can significantly reduce the viscosity of the whole blood of the mice with the melasma and improve the viscosity of the blood; in addition, the activities of the SOD in the livers and skins of the mice of the modeling group with the melasma is obviously decreased when compared with the normal group, and the contents of the MDA and the NO are higher; and after feeding the pharmaceutical composition, the content of the MDA is obviously decreased and the activity of the SOD is increased, which illustrates that ginseng oral liquid for treating the melasma may reduce the formation of the melanin by decreasing the activity of the free radical scavenging enzyme and reducing lipid peroxidation.

One skilled in the art will understand that the embodiment of the present invention as shown in the drawings and described above is exemplary only and not intended to be limiting.

It will thus be seen that the objects of the present invention have been fully and effectively accomplished. Its embodiments have been shown and described for the purposes of illustrating the functional and structural principles of the present invention and is subject to change without departure from such principles.

## Claims

1. A method for preparing a pharmaceutical composition for treating melasma, comprising:
mixing the following four substances for obtaining a mixture, wherein the mixture is the pharmaceutical composition for treating the melasma, wherein the four substances comprises:
A) ethanol extract of ginseng and salvia; B) volatile oil of mint and orange peel; C) aqueous extract of the ginseng, the salvia, the mint, the orange peel, peony, poria, licorice, peach seed, yam and penthorum chienese; D) a fermentation conversion solution of fruits and vegetables;
wherein a method for preparing the A) substance comprises: mixing the ginseng and the salvia, processing reflux extraction on the mixture with an aqueous solution of ethanol for obtaining extract and a first residue, collecting the extract, wherein the extract is the ethanol extract of the ginseng;
wherein a method for preparing the B) substance comprises: immersing the mint and the orange peel in water, processing distillation extraction for obtaining distillate and a second residue, wherein the distillate is the volatile oil of the mint and the orange peel;
wherein a method for preparing the C) substance comprises: mixing the peony, the poria, the licorice, the peach seed, the yam, the penthorum chienese, the first residue and the second residue, boiling, filtering and collecting filtrate, adding absolute ethanol and waiting for collecting supernatant, wherein the supernatant is the aqueous extract of the ginseng, the salvia, the mint, the orange peel, the peony, the poria, the licorice, the peach seed, the yam and the penthorum chienese;
wherein a method for preparing the D) substance comprises steps of: mixing the following fruits and the vegetables: konjac, eggplant, asparagus, spinach, bean sprout, broccoli, cabbage, turnip, cucumber, pea, red pepper, celery, scallion, garlic, grape, grapefruit, watermelon, peach, orange, blueberry, banana, lychee, bitter gourd, leek, pomegranate, dragon fruit, carrot, tomato, Chinese cabbage, celery, bell pepper, lettuce, pear, ginger, taro, bean, pumpkin, lotus root, cherry, kiwi, plum, strawberry, fig, kumquat, citrus, Sounthland pear, melon, cantaloupe, pawpaw, onion, mulberry, sugar beet and lemon, with a bacteria liquid of *Lactobacillus acidophilus,* the bacteria liquid of the *Bifidobacterium longum,* the bacteria liquid of the *Lactobacillus delbrueckii subspbulgaricus* and the bacteria liquid of the *Streptococcus thermophilus,* fermenting for obtaining a fermentation product, wherein the fermentation product is the fermentation conversion solution of fruits and vegetables; and
wherein a mass ratio of the ginseng, the peony, the poria, the licorice, the orange peel, the yam, the mint, the peach seed, the salvia and the penthorum chienese is (1.5-2.5): (1.5-2.5): (1.9-2.9): (0.5-1.5): (0.8-1.7): (1.5-2.5): (0.8-1.7): (0.8-1.7): (1.5-2.5): (3.5-4.5); and
wherein in the methods for preparing the A) substance:
reflux extraction time is 2h-5h, the reflux extraction time is preferably 2h, 3h or 5h;
a concentration of the aqueous solution of ethanol is 70%-95% (according to volume fraction); the concentration is preferably 70%, 90% or 95%;
wherein in the methods for preparing the B) substance:
immersion time is 1min-60min, the immersion time is preferably 1min, 30min or 60min;
a distillation temperature is 90°C-100°C, the distillation temperature is preferably 90°C, 95°C or 100°C.
wherein in the method for preparing the C) substance:
a boiling temperature is 90°C-100°C, the boiling temperature is preferably 90°C, 95°C or 100°C; a boiling time is 1h-2h, the boiling time is preferably 1h or 2h;
a method for adding the absolute ethanol comprises adding the ethanol into the filtrate until a volume percentage of the ethanol in the filtrate is 40%;
wherein in the method for preparing the D) substance:
fermentation temperature is 18°C-37°C, the fermentation temperature is preferably 18°C, 23°C or 37°C, fermentation time is 10 days to 180 days, the fermentation time is preferably 10 days, 15 days or 180 days, a fermentation method comprises stirring while fermenting.

2. The method, as claimed in claim 1, wherein:
the method for preparing the A) substance further comprises steps of:
removing the ethanol from the extract, and then concentrating and drying for obtaining a dried product, wherein the dried product is the ethanol extract of the ginseng and the salvia;
the method for preparing the C) substance further comprises steps of:
removing the ethanol from the supernatant after collecting the supernatant, filtering and collecting the filtrate, and then concentrating and drying for obtaining the dried product, wherein the dried product is the aqueous extract of the ginseng, the salvia, the mint, the orange peel, the peony, the poria, the licorice, the peach seed, the yam and the penthorum chienese;
the method for preparing the D) substance further comprises steps for crushing the fruits and vegetables into 40-50 meshes size before the fermentation;
after the fermentation, the method for preparing the D) substance further comprises steps of: filtering the fermentation product, collecting the filtrate, ultrafiltering, collecting ultrafiltrate, wherein the ultrafiltrate is the fermentation conversion solution of fruits and vegetables.

3. The method, as recited in claim 1 or 2, wherein each fruit or vegetable is of the same weight.

4. The method, as recited in claims 1, 2 or 3, wherein:
in the method, every 1L the mixture is prepared by: mixing (5-50)g the ethanol extract of the ginseng and the salvia, (5-50)g the volatile oil of the mint and the orange peel, (50-200)g the aqueous extract of the ginseng, the salvia, the mint, the orange peel, the peony, the poria, the licorice, the peach seed, the yam and the penthorum chienese and the fermentation conversion solution of the fruits and vegetables, adding the fermentation conversion solution of fruits and vegetables for making up a volume to 1L;
preferably, every 1L the mixture is prepared by: mixing (5, 15 or 50)g the ethanol extract of the ginseng and the salvia, (5, 20 or 50)g the volatile oil of the mint and the orange peel, (50, 160 or 200)g the aqueous extract of the ginseng, the salvia, the mint, the orange peel, the peony, the poria, the licorice, the peach seed, the yam and the penthorum chienese and the fermentation conversion solution of the fruits and vegetables, adding the fermentation conversion solution of fruits and vegetables for making up a volume to 1L;
in the method for preparing the A) substance:
a ratio of the aqueous solution of the ethanol, the ginseng and the salvia is (1-10)ml: 0.5g: 0.5g;
preferably, the ratio of the aqueous solution of the ginseng and the salvia is (1, 5 or 10)ml: 0.5g: 0.5g;
in the method for preparing the B) substance:
the ratio of the mint, the orange peel and the water is (1-10)g: (1-10)g: (1-10)ml;
preferably, the ratio of the mint, the orange peel and the water is (1, 5 or 10)g: (1, 5 or 10)g: (1, 5 or 10)ml;
in the method for preparing the C) substance:
the ratio of the peony, the poria, the licorice, the peach seed, the yam, the penthorum chienese and the water is (1-10)g: (1-10)g: (1-10)g: (1-10)g: (1-10)g: (1-10)g: (10-100)ml;
preferably, the ratio of the peony, the poria, the licorice, the peach seed, the yam, the penthorum chienese and the water is (1, 2 or 10)g: (1, 2.4 or 10)g: (1, 5 or 10)g: (1, 1.2 or 10)g: (1, 2 or 10)g: (1, 4 or 10)g: (50, 60 or 100)ml;
in the method for preparing the D) substance:
the ratio of the bacteria liquid of the *Lactobacillus acidophilus,* the bacteria liquid of the *Bifidobacterium longum,* the bacteria liquid of the *Lactobacillus delbrueckii subsp bulgaricus,* the bacteria liquid of the *Streptococcus thermophilus,* the fermentation conversion solution of the fruits and vegetables and water is (2000-8000)ml: (2000-8000)ml: (2000-8000)ml: (2000-8000)ml: (1000-1500)kg: (1000-1500)kg;
preferably, the ratio of the bacteria liquid of the *Lactobacillus acidophilus,* the bacteria liquid of the *Bifidobacterium longum,* the bacteria liquid of the *Lactobacillus delbrueckii subsp bulgaricus,* the bacteria liquid of the *Streptococcus thermophilus,* the fermentation conversion solution of the fruits and vegetables and the water is (2000, 5000 or 8000)ml: (2000, 5000 or 8000)ml: (2000, 5000 or 8000)ml: (2000, 5000 or 8000)ml: (1000, 1200 or 1500)kg: (1000, 1200 or 1500)kg.

5. The method, as recited in claim 1, 2, 3, or 4, wherein:
a method for preparing the bacteria liquid of the *Lactobacillus acidophilats* comprises: fermenting and growing *Lactobacillus acidophilus* for obtaining the fermentation production, wherein the fermentation production is the bacteria liquid of the *Lactobacillus acidophilus;* wherein the fermentation temperature is 20°C-41°C, the fermentation temperature is preferably 20°C, 37°C or 41°C, the fermentation time is 15h-36h, the fermentation time is preferably 15h, 16h or 36h;
a method for preparing the bacteria liquid of the *Bifidobacterium longum* comprises: fermenting and growing *Bifidobacterium longum* for obtaining the fermentation production, wherein the fermentation production is the bacteria liquid of the *Bifidobacterium longum;* wherein the fermentation temperature is 20°C-41°C, the fermentation temperature is preferably 20°C, 37°C or 41°C, the fermentation time is 15h-36h, the fermentation time is preferably 15h, 16h or 36h;
a method for preparing the bacteria liquid of the *Lactobacillus delbrueckii subsp bulgaricus* comprises: fermenting and growing *Lactobacillus delbrueckii subsp bulgaricus* for obtaining the fermentation production, wherein the fermentation production is the bacteria liquid of the *Lactobacillus delbrueckii subsp bulgaricus;* wherein the fermentation temperature is 20°C-41°C, the fermentation temperature is preferably 20°C, 37°C or 41°C, the fermentation time is 15h-36h, the fermentation time is preferably 15h, 16h or 36h;
a method for preparing the bacteria liquid of the *Streptococcus thermophilus* comprises: fermenting and growing *Streptococcus thermophilus* for obtaining the fermentation production, wherein the fermentation production is the bacteria liquid of the *Streptococcus thermophilus;* wherein the fermentation temperature is 20°C-41°C, the fermentation temperature is preferably 20°C, 37°C or 41°C, the fermentation time is 15h-36h, the fermentation time is preferably 15h, 16h or 36h.

6. The method, as recited in claim 1, 2, 3, 4 or 5, wherein:
the *Lactobacillus acidophilus* is the *Lactobacillus acidophilus* CGMCC (China General Microbiological Culture Collection) 1.1854, the *Bifidobactreium longum* is the *Bifidobacterium longum* CGMCC 1.2186, the *Lactobacillus delbrueckii subsp bulgaricus* is the *Lactobacillus delbrueckii subsp bulgaricus* CGMCC 1.1480, the *Streptococcus thermophilus* is the *Streptococcus thermophilus* CGMCC 1.2471.

7. A pharmaceutical composition for treating melasma prepared by the method recited in claim 1, 2, 3, 4, 5 or 6.

8. A pharmaceutical composition according to claim 7 for use in treating melasma.

## Patentansprüche

1. Verfahren zur Zubereitung einer pharmazeutischen Zusammensetzung zur Behandlung von Melasma, umfassend:
Mischen der folgenden vier Substanzen zum Erhalt eines Gemischs, wobei das Gemisch die pharmazeutische Zusammensetzung zur Behandlung des Melasmas ist, worin die vier Substanzen umfassen:
A) Ethanolextrakt aus Ginseng und Salbei; B) flüchtiges Öl von Minze und Orangenschale; C) wässriger Extrakt aus dem Ginseng, dem Salbei, der Minze, der Orangenschale, von Pfingstrose, Poria, Süßholz, Pfirsichsamen, Yamswurzel und Penthorum chinense; D) eine Fermentationsumwandlungslösung von Früchten und Gemüsen;
wobei ein Verfahren zur Zubereitung der A)-Substanz umfasst: Mischen des Ginsengs und des Salbeis, Prozessieren einer Rücklaufextraktion an dem Gemisch mit einer wässrigen Lösung von Ethanol zum Erhalt des Extrakts und eines ersten Rückstands, Sammeln des Extrakts, wobei der Extrakt der Ethanolextrakt des Ginsengs ist;
wobei ein Verfahren zur Zubereitung der B)-Substanz umfasst: Eintauchen der Minze und der Orangenschale in Wasser, Prozessieren einer Destillationsextraktion zum Erhalt des Destillats und eines zweiten Rückstands, wobei das Destillat das flüchtige Öl der Minze und der Orangenschale ist;
wobei ein Verfahren zur Zubereitung der C)-Substanz umfasst: Mischen der Pfingstrose, der Poria, des Süßholzes, der Pfirsichsamen, der Yamswurzel, des Penthorum chinense, des ersten Rückstands und des zweiten Rückstands, Aufkochen, Filtrieren und Sammeln des Filtrats, Zugeben von absolutem Ethanol und Warten zum Sammeln des Überstands, wobei der Überstand der wässrige Extrakt des Ginsengs, des Salbeis, der Minze, der Orangenschale, der Pfingstrose, der Poria, des Süßholzes, der Pfirsichsamen, der Yamswurzel und des Penthorum chinense ist;
wobei ein Verfahren zur Zubereitung der D)-Substanz die folgenden Schritte umfasst:
Mischen der folgenden Früchte und Gemüse: Konjak, Aubergine, Spargel, Spinat, Bohnensprossen, Broccoli, Kohl, Steckrübe, Gurke, Erbse, rote Paprika, Sellerie, Schalotte, Knoblauch, Traube, Grapefruit, Wassermelone, Pfirsich, Orange, Blaubeere, Banane, Lychee, Bitterkurbis, Lauch, Granatapfel, Drachenfrucht, Karotte, Tomate, Chinakohl, Sellerie, Paprikaschote, Kopfsalat, Birne, Ingwer, Taro, Bohne, Kürbis, Lotuswurzel, Kirsche, Kiwi, Pflaume, Erdbeere, Feige, Kumquat, Zitrusfrucht, Southland-Birne, Melone, Cantaloupe-Melone, Papaya, Zwiebel, Maulbeere, Zuckerrübe und Limone, mit einer Bakterienflüssigkeit von *Lactobacillus acidophilus,* der Bakterienflüssigkeit von *Bifidobacterium longum,* der Bakterienflüssigkeit von *Lactobacillus delbrueckii subsp.*
*bulgaricus* und der Bakterienflüssigkeit von *Streptococcus thermophilus,* Fermentieren zum Erhalt eines Fermentationsprodukts, wobei das Fermentationsprodukt die Fermentationsumwandlungslösung von Früchten und Gemüsen ist; und
wobei ein Massenverhältnis des Ginsengs, der Pfingstrose, der Poria, des Süßholzes, der Orangenschale, der Yamswurzel, der Minze, der Pfirsichsamen, des Salbei und des Penthorum chinense (1,5-2,5) : (1,5-2,5): (1,9-2,9) : (0,5-1,5): (0,8-1,7): (1,5-2,5): (0,8-1,7) : (0,8-1,7) : (1,5-2,5) : (3,5-4,5) beträgt; und
wobei bei den Verfahren zur Zubereitung der A)-Substanz:
die Rücklaufextraktionszeit 2 h-5 h beträgt, wobei die Rücklaufextraktionszeit vorzugsweise 2 h, 3 h oder 5 h beträgt;
eine Konzentration der wässrigen Lösung des Ethanols 70%-95% (nach Volumenfraktion) beträgt; wobei die Konzentration vorzugsweise 70%, 90% oder 95% beträgt;
wobei bei den Verfahren zur Zubereitung der B)-Substanz:
die Eintauchzeit 1 min-60 min beträgt, wobei die Eintauchzeit vorzugsweise 1 min, 30 min oder 60 min beträgt;
die Destillationstemperatur 90°C-100°C beträgt, wobei die Destillationstemperatur vorzugsweise 90°C, 95°C oder 100°C beträgt;
wobei bei den Verfahren zur Zubereitung der C)-Substanz:
eine Siedetemperatur 90°C-100°C beträgt, wobei die Siedetemperatur vorzugsweise 90°C, 95°C oder 100°C beträgt; eine Siedezeit 1 h-2 h beträgt, wobei die Siedezeit vorzugsweise 1 h oder 2 h beträgt;
ein Verfahren zum Zugeben des absoluten Ethanols das Zugeben des Ethanols in das Filtrat umfasst, bis ein prozentualer Volumenanteil des Ethanols in dem Filtrat 40% beträgt;
wobei bei dem Verfahren zur Zubereitung der D)-Substanz:
die Fermentationstemperatur 18°C-37°C beträgt, wobei die Fermentationstemperatur vorzugsweise 18°C, 23°C oder 37°C beträgt, die Fermentationszeit 10 Tage bis 180 Tage beträgt, wobei die Fermentationszeit vorzugsweise 10 Tage, 15 Tage oder 180 Tage beträgt, und ein Fermentationsverfahren das Rühren während des Fermentierens umfasst.

2. Verfahren, wie in Anspruch 1 beansprucht, wobei:
das Verfahren zur Zubereitung der A)-Substanz außerdem die folgenden Schritte umfasst:
Entfernen des Ethanols aus dem Extrakt, und dann Konzentrieren und Trocknen zum Erhalt eines getrockneten Produkts, wobei das getrocknete Produkt der Ethanolextrakt aus dem Ginseng und dem Salbei ist;
das Verfahren zur Zubereitung der C)-Substanz außerdem die folgenden Schritte umfasst:
Entfernen des Ethanols aus dem Überstand nach dem Absammeln des Überstands, Filtrieren und Sammeln des Filtrats, und dann Konzentrieren und Trocknen zum Erhalt des getrockneten Produkts, wobei das getrocknete Produkt der wässrige Extrakt aus dem Ginseng, dem Salbei, der Minze, der Orangenschale, der Pfingstrose, der Poria, dem Süßholz, den Pfirsichsamen, der Yamswurzel und dem Penthorum chinense ist;
das Verfahren zur Zubereitung der D)-Substanz außerdem Schritte zum Zerquetschen der Früchte und Gemüse zu einer Größe von 40-50 mesh vor der Fermentation umfasst;
nach der Fermentation, das Verfahren zur Zubereitung der D)-Substanz außerdem die folgenden Schritte umfasst: Filtrieren des Fermentationsprodukts, Sammeln des Filtrats, Ultrafiltrieren, Sammeln des Ultrafiltrats, wobei das Ultrafiltrat die Fermentationsumwandlungslösung der Früchte und Gemüse ist.

3. Verfahren, wie in Anspruch 1 oder 2 rezitiert, wobei jeder Frucht- oder Gemüseanteil von demselben Gewicht ist.

4. Verfahren, wie in Ansprüchen 1, 2 oder 3 rezitiert, wobei:
bei dem Verfahren, jeder 1 Liter des Gemischs zubereitet wird durch: Mischen von (5-50) g des Ethanolextrakts aus dem Ginseng und dem Salbei, (5-50) g des flüchtigen Öls von der Minze und der Orangenschale, (50-200) g des wässrigen Extrakts aus dem Ginseng, dem Salbei, der Minze, der Orangenschale, der Pfingstrose, der Poria, dem Süßholz, den Pfirsichsamen, der Yamswurzel und dem Penthorum chinense und der Fermentationsumwandlungslösung der Früchte und Gemüse, Zugeben der Fermentationsumwandlungslösung der Früchte und Gemüse zum Auffüllen auf ein Volumen von 1 L;
vorzugsweise, jeder 1 Liter des Gemischs zubereitet wird durch: Mischen von (5, 15 oder 50) g des Ethanolextrakts aus dem Ginseng und dem Salbei, (5, 20 oder 50) g des flüchtigen Öls von der Minze und der Orangenschale, (50, 160 oder 200) g des wässrigen Extrakts aus dem Ginseng, dem Salbei, der Minze, der Orangenschale, der Pfingstrose, der Poria, dem Süßholz, den Pfirsichsamen, der Yamswurzel und dem Penthorum chinense und der Fermentationsumwandlungslösung der Früchte und Gemüse, Zugeben der Fermentationsumwandlungslösung der Früchte und Gemüse zum Auffüllen auf ein Volumen von 1 L;
bei dem Verfahren zur Zubereitung der A)-Substanz:
ein Verhältnis von der wässrigen Lösung des Ethanols, dem Ginseng und dem Salbei (1-10) ml : 0,5 g : 0,5 g beträgt;
vorzugsweise, das Verhältnis von der wässrigen Lösung, dem Ginseng und dem Salbei (1, 5 oder 10) ml : 0,5 g : 0,5 g beträgt;
bei dem Verfahren zur Zubereitung der B)-Substanz:
das Verhältnis von der Minze, der Orangenschale und dem Wasser (1-10) g : (1-10) g : (1-10) ml beträgt;
vorzugsweise, das Verhältnis von der Minze, der Orangenschale und dem Wasser (1, 5 oder 10) g : (1, 5 oder 10) g : (1, 5 oder 10) ml beträgt;
bei dem Verfahren zur Zubereitung der C)-Substanz:
das Verhältnis von der Pfingstrose, der Poria, dem Süßholz, den Pfirsichsamen, der Yamswurzel, dem Penthorum chinense und dem Wasser (1-10) g : (1-10) g : (1-10) g : (1-10) g : (1-10) g : (1-10) g : (10-100) ml beträgt;
vorzugsweise, das Verhältnis von der Pfingstrose, der Poria, dem Süßholz, den Pfirsichsamen, der Yamswurzel, dem Penthorum chinense und dem Wasser (1, 2 oder 10) g : (1, 2,4 oder 10) g : (1, 5 oder 10) g : (1, 1,2 oder 10) g : (1, 2 oder 10) g : (1, 4 oder 10) g : (50, 60 oder 100) ml beträgt;
bei dem Verfahren zur Zubereitung der D)-Substanz:
das Verhältnis von der Bakterienflüssigkeit von *Lactobacillus acidophilus,* der Bakterienflüssigkeit von *Bifidobacterium longum,* der Bakterienflüssigkeit von *Lactobacillus delbrueckii subsp. bulgaricus* und der Bakterienflüssigkeit von *Streptococcus thermophilus,* der Fermentationsumwandlungslösung der Früchte und Gemüse und von Wasser (2000-8000) ml : (2000-8000) ml : (2000-8000) ml : (2000-8000) ml : (1000-1500) kg : (1000-1500) kg beträgt;
vorzugsweise, das Verhältnis von der Bakterienflüssigkeit von *Lactobacillus acidophilus,* der Bakterienflüssigkeit von *Bifidobacterium longum,* der Bakterienflüssigkeit von *Lactobacillus delbrueckii subsp. Bulgaricus,* der Bakterienflüssigkeit von *Streptococcus thermophilus,* der Fermentationsumwandlungslösung der Früchte und Gemüse und von Wasser (2000, 5000 oder 8000) ml : (2000, 5000 oder 8000) ml : (2000, 5000 oder 8000) ml : (2000, 5000 oder 8000) ml : (1000, 1200 oder 1500) kg : (1000, 1200 oder 1500) kg beträgt.

5. Verfahren, wie in Anspruch 1, 2, 3 oder 4 rezitiert, wobei:
ein Verfahren zur Zubereitung der Bakterienflüssigkeit von *Lactobacillus acidophilus* umfasst: Fermentieren und Züchten von *Lactobacillus acidophilus* zum Erhalt des Fermentationsprodukts, wobei das Fermentationsprodukt die Bakterienflüssigkeit des *Lactobacillus acidophilus* ist; wobei die Fermentationstemperatur 20°C-41°C beträgt, wobei die Fermentationstemperatur vorzugsweise 20°C, 37°C oder 41 °C beträgt, die Fermentationszeit 15 h-36 h beträgt, wobei die Fermentationszeit vorzugsweise 15 h, 16 h oder 36 h beträgt;
ein Verfahren zur Zubereitung der Bakterienflüssigkeit von *Bifidobacterium longum* umfasst: Fermentieren und Züchten von *Bifidobacterium longum* zum Erhalt des Fermentationsprodukts, wobei das Fermentationsprodukt die Bakterienflüssigkeit von *Bifidobacterium longum* ist; wobei die Fermentationstemperatur 20°C-41°C beträgt, wobei die Fermentationstemperatur vorzugsweise 20°C, 37°C oder 41 °C beträgt, die Fermentationszeit 15 h-36 h beträgt, wobei die Fermentationszeit vorzugsweise 15 h, 16 h oder 36 h beträgt;
ein Verfahren zur Zubereitung der Bakterienflüssigkeit von *Lactobacillus delbrueckii subsp. bulgaricus* umfasst: Fermentieren und Züchten von *Lactobacillus delbrueckii subsp. bulgaricus* zum Erhalt des Fermentationsprodukts, wobei das Fermentationsprodukt die Bakterienflüssigkeit von *Lactobacillus delbrueckii subsp. bulgaricus* ist; wobei die Fermentationstemperatur 20°C-41°C beträgt, wobei die Fermentationstemperatur vorzugsweise 20°C, 37°C oder 41 °C beträgt, die Fermentationszeit 15 h-36 h beträgt, wobei die Fermentationszeit vorzugsweise 15 h, 16 h oder 36 h beträgt;
ein Verfahren zur Zubereitung der Bakterienflüssigkeit von *Streptococcus thermophilus* umfasst: Fermentieren und Züchten von *Streptococcus thermophilus* zum Erhalt des Fermentationsprodukts, wobei das Fermentationsprodukt die Bakterienflüssigkeit von *Streptococcus thermophilus* ist; wobei die Fermentationstemperatur 20°C-41°C beträgt, wobei die Fermentationstemperatur vorzugsweise 20°C, 37°C oder 41 °C beträgt, die Fermentationszeit 15h-36h beträgt, wobei die Fermentationszeit vorzugsweise 15h, 16h oder 36h beträgt.

6. Verfahren, wie in Anspruch 1, 2, 3, 4 oder 5 rezitiert, wobei:
der *Lactobacillus acidophilus* der *Lactobacillus acidophilus* CGMCC (China General Microbiological Culture Collection) 1.1854, das *Bifidobacterium longum* das *Bifidobacterium longum* CGMCC 1.2186 ist, das *Lactobacillus delbrueckii subsp. bulgaricus* das *Lactobacillus delbrueckii subsp. bulgaricus* CGMCC 1.1480 ist, der *Streptococcus thermophilus* der *Streptococcus thermophilus* CGMCC 1.2471 ist.

7. Pharmazeutische Zusammensetzung zur Behandlung von Melasma, die mittels des in Anspruch 1, 2, 3, 4, 5 oder 6 rezitierten Verfahrens zubereitet ist.

8. Pharmazeutische Zusammensetzung gemäß Anspruch 7 zur Verwendung in der Behandlung von Melasma.

## Revendications

1. Procédé pour préparer une composition pharmaceutique pour traiter le mélasme, comprenant :
le mélangeage des quatre substances suivantes pour obtenir un mélange, dans lequel le mélange est la composition pharmaceutique pour traiter le mélasme, dans lequel les quatre substances comprennent :
A) de l'extrait d'éthanol de ginseng et de sauge sclarée ; B) de l'huile volatile de menthe et de pelure d'orange ; C) de l'extrait aqueux du ginseng, de la sauge sclarée, de la menthe, de la pelure orange, de la pivoine, du poria, de la réglisse, du noyau de pêche, de l'igname et du penthorum chinense ; D) une solution de conversion de fermentation de fruits et de légumes ;
dans lequel un procédé pour préparer la substance A) comprend : le mélange du ginseng et de la sauge sclarée, un traitement d'extraction par reflux sur le mélange avec une solution aqueuse d'éthanol pour obtenir un extrait et un premier résidu, la collecte de l'extrait, dans lequel l'extrait est l'extrait d'éthanol du ginseng ;
dans lequel un procédé pour préparer la substance B) comprend : l'immersion de la menthe et de la pelure d'orange dans de l'eau, un traitement d'extraction par distillation pour obtenir un distillat et un second résidu, dans lequel le distillat est l'huile volatile de la menthe et de la pelure d'orange ;
dans lequel un procédé pour préparer la substance C) comprend : le mélange de la pivoine, du poria, de la réglisse, du noyau de pêche, de l'igname, du penthorum chinense, le premier résidu et le second résidu, l'ébullition, le filtrage et la collecte de filtrat, l'ajout d'éthanol absolu et l'attente de la collecte du surnageant, dans lequel le surnageant est de l'extrait aqueux du ginseng, de la sauge sclarée, de la menthe, de la pelure orange, de la pivoine, du poria, de la réglisse, du noyau de pêche, de l'igname et du penthorum chinense ;
dans lequel un procédé pour préparer la substance D) comprend les étapes de : mélange des fruits et des légumes suivants : konjac, aubergine, asperge, épinard, germe de haricot, brocoli, chou, navet, concombre, pois, poivron rouge, céleri, oignon, ail, raisin, pamplemousse, pastèque, pêche, orange, myrtille, banane, litchi, margose, poireau, grenade, fruit du dragon, carotte, tomate, chou chinois, céleri, poivron d'Amérique, laitue, poire, gingembre, taro, haricot, potiron, racine de lotus, cerise, kiwi, prune, fraise, figue, kumquat, agrumes, poire de Nouvelle Zélande, melon, cantaloup, papaye, oignon, mûre, betterave à sucre et citron, avec un liquide bactérien de *Lactobacillus acidophilus,* le liquide bactérien de *Bifidobacterium longum,* le liquide bactérien de *Lactobacillus delbrueckii subsp. bulgaricus* et le liquide bactérien de *Streptococcus thermophilus,* fermentation pour obtenir un produit de fermentation, dans lequel le produit de fermentation est la solution de conversion de fermentation de fruits et légumes ; et
dans lequel un rapport de masse du ginseng, de la pivoine, du poria, de la réglisse, de la pelure orange, de l'igname, de la menthe, du noyau de pêche, de la sauge sclarée et du penthorum chinense sont (1,5-2,5) : (1,5-2,5) : (1,9-2,9) : (0,5-1,5) : (0,8-1,7) : (1, 5-2,5) : (0,8-1,7) : (0,8-1,7) : (1,5-2,5) : (3,5-4,5) ; et
dans lequel dans les procédés pour préparer la substance A) :
le temps d'extraction par reflux est 2 h à 5 h, le temps d'extraction par reflux est de préférence 2 h, 3 h ou 5 h ;
une concentration de la solution aqueuse d'éthanol est 70 % à 95 % (selon la fraction volumique) ; la concentration est de préférence de 70 %, 90 % ou 95 %;
dans lequel dans les procédés pour préparer la substance B) :
le temps d'immersion est 1 min à 60 min, le temps d'immersion est de préférence 1 min, 30 min ou 60 min ;
une température de distillation est 90 °C à 100 °C, la température de distillation est de préférence 90 °C, 95 °C ou 100 °C,
dans lequel dans le procédé pour préparer la substance C) :
une température d'ébullition est 90 °C à 100 °C, la température d'ébullition est de préférence 90 °C, 95 °C ou 100 °C ; un temps d'ébullition est 1 h à 2 h, le temps d'ébullition est de préférence 1 h ou 2 h ;
un procédé pour ajouter l'éthanol absolu comprend l'ajout de l'éthanol dans le filtrat jusqu'à ce qu'un pourcentage volumique de l'éthanol dans le filtrat soit de 40 % ;
dans lequel dans le procédé pour préparer la substance D) :
la température de fermentation est 18 °C à 37 °C, la température de fermentation est de préférence 18 °C, 23 °C ou 37 °C, le temps de fermentation est 10 jours à 180 jours, le temps de fermentation est de préférence 10 jours, 15 jours ou 180 jours, un procédé de fermentation comprend de l'agitation tout en faisant fermenter.

2. Procédé, selon la revendication 1, dans lequel :
le procédé pour préparer la substance A) comprend en outre les étapes de :
enlèvement de l'éthanol depuis l'extrait et ensuite concentration et séchage pour obtenir un produit séché, dans lequel le produit séché est l'extrait d'éthanol du ginseng et de la sauge sclarée ;
le procédé pour préparer la substance C) comprend en outre les étapes de :
enlèvement de l'éthanol depuis le surnageant après la collecte du surnageant, le filtrage et la collecte du filtrat, et ensuite la concentration et le séchage pour obtenir le produit séché, dans lequel le produit séché est l'extrait aqueux du ginseng, de la sauge sclarée, de la menthe, de la pelure orange, de la pivoine, du poria, de la réglisse, du noyau de pêche, de l'igname et du penthorum chinense ;
le procédé pour préparer la substance D) comprend en outre des étapes pour écraser les fruits et les légumes dans des tamis de taille 40 à 50 avant la fermentation ;
après la fermentation, le procédé pour préparer la substance D) comprend en outre les étapes de : filtrage du produit de fermentation, collecte du filtrat, ultrafiltrage, collecte de l'ultrafiltrat, dans lequel l'ultrafiltrat est la solution de conversion de fermentation de fruits et légumes.

3. Procédé, selon la revendication 1 ou 2, dans lequel chaque fruit ou légume a le même poids.

4. Procédé, selon les revendications 1, 2 ou 3, dans lequel :
dans le procédé, chaque 1 L de mélange est préparé par : mélange (5 à 50) g de l'extrait d'éthanol du ginseng et de la sauge sclarée, (5 à 50) g de l'huile volatile de la menthe et de la pelure orange, (50 à 200) g de l'extrait aqueux du ginseng, de la sauge sclarée, de la menthe, de la pelure orange, de la pivoine, du poria, de la réglisse, du noyau de pêche, de l'igname et du penthorum chinense et la solution de conversion de fermentation des fruits et légumes, l'ajout de la solution de conversion de fermentation de fruits et légumes pour composer un volume à 1 L ;
de préférence, chaque 1 L de mélange est préparé par : mélange (5, 15 ou 50) g de l'extrait d'éthanol du ginseng et de la sauge sclarée, (5, 20 ou 50) g de l'huile volatile de la menthe et de la pelure orange, (50, 160 ou 200) g de l'extrait aqueux du ginseng, de la sauge sclarée, de la menthe, de la pelure orange, de la pivoine, du poria, de la réglisse, du noyau de pêche, de l'igname et du penthorum chinense et la solution de conversion de fermentation des fruits et des légumes, ajout de la solution de conversion de fermentation de fruits et légumes pour composer un volume à 1 La ;
dans le procédé pour préparer la substance A) :
un rapport de la solution aqueuse de l'éthanol, du ginseng et de la sauge sclarée est de (1 à 10) ml : 0,5 g : 0,5 g ;
de préférence, le rapport de la solution aqueuse du ginseng et de la sauge sclarée est (1, 5 ou 10) ml : 0,5 g : 0,5 g ;
dans le procédé pour préparer la substance B) :
le rapport de la menthe, de la pelure d'orange et de l'eau est (1-10) g : (1-10) g : (1-10) ml ;
de préférence, le rapport de la menthe, de la pelure orange et de l'eau est (1, 5 ou 10) g : (1, 5 ou 10) g : (1, 5 ou 10) ml ;
dans le procédé pour préparer la substance C) :
le rapport de la pivoine, du poria, de la réglisse, du noyau de pêche, de l'igname, du penthorum chinense et de l'eau est (1-10) g : (1-10) g : (1-10) g : (1-10) g : (1-10) g : (1-10) g : (10-100) ml ;
de préférence, le rapport de la pivoine, du poria, de la réglisse, du noyau de pêche, de l'igname, du penthorum chinense et de l'eau est (1, 2 ou 10) g : (1, 2,4 ou 10) g : (1, 5 ou 10) g : (1, 1,2 ou 10) g : (1, 2 ou 10) g : (1, 4 ou 10) g : (50, 60 ou 100) ml ;
dans le procédé pour préparer la substance D) :
le rapport du liquide bactérien de *Lactobacillus acidophilus,* du liquide bactérien de *Bifidobacterium longum,* du liquide bactérien de *Lactobacillus delbrueckii subsp. bulgaricus,* le liquide bactérien de *Streptococcus thermophilus,* la solution de conversion de fermentation des fruits et légumes et de l'eau est (2 000-8 000) ml : (2 000-8 000) ml : (2 000-8 000) ml : (2 000-8 000) ml : (1 000-1 500) kg : (1 000-1 500) kg ;
de préférence, le rapport du liquide bactérien de *Lactobacillus acidophilus,* du liquide bactérien de *Bifidobacterium longum,* du liquide bactérien de *Lactobacillus delbrueckii subsp. bulgaricus,* du liquide bactérien de *Streptococcus thermophilus,* la solution de conversion de fermentation des fruits et légumes et de l'eau est (2 000, 5 000 ou 8 000) ml : (2 000, 5 000 ou 8 000) ml : (2 000, 5 000 ou 8 000) ml : (2 000, 5 000 ou 8 000) ml : (1 000, 1 200 ou 1 500) kg : (1 000, 1 200 ou 1 500) kg.

5. Procédé, selon la revendication 1, 2, 3, ou 4, dans lequel :
un procédé pour préparer le liquide bactérien de *Lactobacillus acidophilus* comprend : la fermentation et la croissance de *Lactobacillus acidophilus* pour obtenir la production de fermentation, dans lequel la production de fermentation est le liquide bactérien de *Lactobacillus acidophilus ;* dans lequel la température de fermentation est 20 °C à 41 °C, la température de fermentation est de préférence 20 °C, 37 °C ou 41 °C, le temps de fermentation est 15 h à 36 h, le temps de fermentation est de préférence 15 h, 16 h ou 36 h ;
un procédé pour préparer le liquide bactérien de *Bifidobacterium longum* comprend : la fermentation et la croissance de *Bifidobacterium longum* pour obtenir la production de fermentation, dans lequel la production de fermentation est le liquide bactérien de *Bifidobacterium longum ;* dans lequel la température de fermentation est 20 °C à 41 °C, la température de fermentation est de préférence 20 °C, 37 °C ou 41 °C, le temps de fermentation est 15 h à 36 h, le temps de fermentation est de préférence 15 h, 16 h ou 36 h ;
un procédé pour préparer le liquide bactérien de *Lactobacillus delbrueckii subsp. bulgaricus* comprend :
la fermentation et la croissance de *Lactobacillus delbrueckii subsp. bulgaricus* pour obtenir la production de fermentation, dans lequel la production de fermentation est le liquide bactérien de *Lactobacillus delbrueckii subsp. bulgaricus ;* dans lequel la température de fermentation est 20 °C à 41 °C, la température de fermentation est de préférence 20 °C, 37 °C ou 41 °C, le temps de fermentation est 15 h à 36 h, le temps de fermentation est de préférence 15 h, 16 h ou 36 h ;
un procédé pour préparer le liquide bactérien de *Streptococcus thermophilus* comprend : la fermentation et la croissance de *Streptococcus thermophilus* pour obtenir la production de fermentation, dans lequel la production de fermentation est le liquide bactérien de *Streptococcus thermophilus ;* dans lequel la température de fermentation est 20 °C à 41 °C, la température de fermentation est de préférence 20 °C, 37 °C ou 41 °C, le temps de fermentation est 15 h à 36 h, le temps de fermentation est de préférence 15 h, 16 h ou 36 h.

6. Procédé, selon la revendication 1, 2, 3, 4 ou 5, dans lequel :
le *Lactobacillus acidophilus* est *Lactobacillus acidophilus* CGMCC (China General Microbiological Culture Collection) 1.1854, le *Bifidobacterium longum* est *Bifidobacterium longum* CGMCC 1.2186, *Lactobacillus delbrueckii subsp. bulgaricus* est *Lactobacillus delbrueckii subsp. bulgaricus* CGMCC 1.1480, le *Streptococcus thermophilus* est *Streptococcus thermophilus* CGMCC 1.2471.

7. Composition pharmaceutique pour traiter le mélasme préparé par le procédé selon la revendication 1, 2, 3, 4, 5 ou 6.

8. Composition pharmaceutique selon la revendication 7 à utiliser dans traitement du mélasme.
